# EUROPEAN PATENT SPECIFICATION

(11) **EP 1 478 362 B1**
(45) Date of publication and mention of the grant of the patent: **12.10.2005**
(21) Application number: 03700828.1
(22) Date of filing: 31.01.2003
(51) Int. Cl.: A61K 31/50, A61K 38/48, A61K 38/49, A61P 9/10

(54) **A COMBINATION TREATMENT FOR ACUTE MYOCARDIAL INFARCTION**
KOMBINATIONSBEHANDLUNG BEI AKUTEM MYOKARDINFARKT
POLYTH RAPIE CONTRE L'INFARCTUS AIGU DU MYOCARDE

(30) Priority: 01.02.2002 FI 20020197
(43) Date of publication of application: 24.11.2004
(73) Proprietor: Orion Corporation, 02200 Espoo (FI)
(72) Inventor: PÖDER, Pentti, FIN-02210 Espoo (FI)
(74) Representative: Sexton, Jane Helen
(86) International application number: PCT/FI2003/000078
(87) International publication number: WO 2003/063870

(56) References cited:
- GERSH B J: "Current issues in reperfusion therapy." THE AMERICAN JOURNAL OF CARDIOLOGY. UNITED STATES 22 OCT 1998, vol. 82, no. 8B, 22 October 1998 (1998-10-22), pages 3P-11P, XP002242596 ISSN: 0002-9149
- KERSTEN JUDY R ET AL: "Levosimendan, a new positive inotropic drug, decreases myocardial infarct size via activation of KATP channels." ANESTHESIA & ANALGESIA, vol. 90, no. 1, January 2000 (2000-01), pages 5-11, XP002242597 ISSN: 0003-2999
- LEHTONEN L A: "Levosimendan: a parenteral calcium-sensitising drug with additional vasodilatory properties." EXPERT OPINION ON INVESTIGATIONAL DRUGS. ENGLAND MAY 2001, vol. 10, no. 5, May 2001 (2001-05), pages 955-970, XP002242598 ISSN: 1354-3784
- GOMES U C ET AL: "Heart failure update." EUROPEAN JOURNAL OF HEART FAILURE: JOURNAL OF THE WORKING GROUP ON HEART FAILURE OF THE EUROPEAN SOCIETY OF CARDIOLOGY. NETHERLANDS AUG 1999, vol. 1, no. 3, August 1999 (1999-08), pages 301-302, XP002242599 ISSN: 1388-9842
- "Vasodilators and inotropes provide symptomatic relief for decompensated heart failure" DRUGS & THERAPY PERSPECTIVES, vol. 18, no. 5, 2002, pages 6-9, XP002242600
- LAM X M ET AL: "Stability and activity of alteplase with injectable drugs commonly used in cardiac therapy." AMERICAN JOURNAL OF HEALTH-SYSTEM PHARMACY: AJHP: OFFICIAL JOURNAL OF THE AMERICAN SOCIETY OF HEALTH-SYSTEM PHARMACISTS. UNITED STATES 1 SEP 1995, vol. 52, no. 17, 1 September 1995 (1995-09-01), pages 1904-1909, XP002242601 ISSN: 1079-2082

## Description

### Technical field

The present invention relates to the use of a synergistic combination of a thrombolytic agent and levosimendan or a pharmaceutically acceptable salt thereof for treating myocardial infarction. The invention also relates to a medical product comprising a thrombolytic agent and levosimendan or a pharmaceutically acceptable salt thereof as a combined preparation.

### Background of the invention

Myocardial infarction is an important complication of coronary artery disease and usually results from a critical reduction in coronary blood flow secondary to coronary thrombosis. Intravenous thrombolytic agent therapy has been widely used to restore flow to the occluded coronary artery. A thrombolytic agent is a medicament capable of lysing the fibrin-platelet thrombus, and thereby permitting blood to again flow through the affected blood vessel. Such agents include streptokinase, urokinase, prourokinase, reteplase, alteplase and tissue-type plasminogen activator (t-PA). The mortality of patients with acute myocardial infarction even if treated with thrombolytic agents remains high.

Levosimendan, which is the (-)-enantiomer of [[4-(1,4,5,6-tetrahydro-4-methyl-6-oxo-3-pyridazinyl)phenyl]hydrazono]propanedinitrile, and the method for its preparation is described in EP 565546 B1. Levosimendan is potent in the treatment of heart failure and has significant calcium dependent binding to troponin. Levosimendan is represented by the formula:

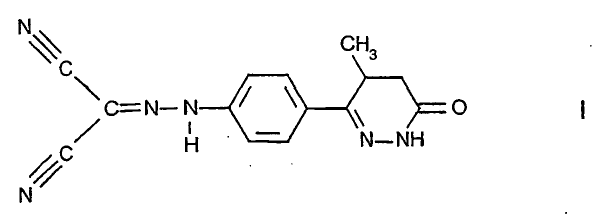

The hemodynamic effects of levosimendan in man are described in Sundberg, S. et al., Am. J. Cardiol., 1995; 75: 1061-1066 and in Lilleberg, J. et al., J. Cardiovasc. Phannacol., 26(Suppl.1), S63-S69, 1995. Pharmacokinetics of levosimendan in man after i.v. and oral dosing is described in Sandell, E.-P. et al., J. Cardiovasc. Pharmacol., 26(Suppl.1), S57-S62, 1995. The use oflevosimendan in the treatment of myocardial ischemia is described in WO 93/21921. The use of levosimendan in the treatment of pulmonary hypertension is described in WO 99/66912. The use of levosimendan in the treatment or prevention of coronary graft vasospasm in described in WO 01/ 00211. Levosimendan solutions suitable for intravenous use have been described in WO 01/19334. Clinical studies have confirmed the beneficial effects of levosimendan in congestive heart failure patients.

### Summary of the invention

It has now been found that administration of a thrombolytic agent together with levosimendan has a beneficial synergistic effect on the mortality of patients treated for myocardial infarction. Therefore, the combination is particularly useful for the treatment of acute myocardial infarction.

Thus, in one aspect the present invention provides the use of an effective amount of a thrombolytic agent and levosimendan or a pharmaceutically acceptable salt thereof for the treatment of acute myocardial infarction, which comprises the simultaneous, separate or sequential administration.

In another aspect the present invention provides the use of a thrombolytic agent in combination with levosimendan or a pharmaceutically acceptable salt thereof for the treatment of acute myocardial infarction.

In another aspect the present invention provides the use of an effective amount of a thrombolytic agent and levosimendan or a pharmaceutically acceptable salt thereof for reducing mortality of patients with acute myocardial infarction, which comprises the simultaneous, separate or sequential administration.

In another aspect the invention provides the use of a thrombolytic agent in combination with levosimendan or a pharmaceutically acceptable salt thereof for reducing mortality of patients with acute myocardial infarction.

In another aspect the invention provides a medical product comprising, separately or together, as active ingredients a thrombolytic agent and levosimendan or a pharmaceutically acceptable salt thereof as a combined preparation.

In another aspect invention provides a pharmaceutical composition comprising as active ingredients a thrombolytic agent and levosimendan or a pharmaceutically acceptable salt thereof.

In another aspect the invention provides the use of a thrombolytic agent and levosimendan or a pharmaceutically acceptable salt thereof as active ingredients in the manufacture of a combined preparation for simultaneous, separate or sequential administration.

In still another aspect the invention provides use of a thrombolytic agent and levosimendan or a pharmaceutically acceptable salt thereof as active ingredients in the manufacture of a medicament for reducing mortality of patients with myocardial infarction.

### Detailed description of the invention

The use of the invention relates to a combination therapy for more effective treatment of acute myocardial infarction. The present method provides a treatment, which improves the overall clinical outcome of patients treated with a thrombolytic agent by administering levosimendan in combination with a thrombolytic agent. In particular, the combination treatment of the invention is able to synergistically reduce mortality of acute myocardial infarction patients.

The terms "treatment" or "treating" as used herein refers to reduction in severity or frequency of symptoms and/or their underlying cause, improving the clinical outcome of a patient, prevention of the occurrence of the symptoms and/or their underlying cause, and improvement or remediation of damage.

The term "acute myocardial infarction" is defined as immediate or sudden (not chronic) infarction of the heart muscle, i.e. an insufficiency of arterial blood flow as a result of occlusion of a coronary artery due to at least partial blockage of the artery by an embolus or thrombus. As used herein, "thrombus" or "embolus" refer to a blood clot within the blood vessel. "At least partial" blockage of the artery means that the artery contains an embolus or thrombus, which reduces the cross sectional area of the artery.

The term "thrombolytic agent" is meant to refer to any agent effective in helping to dissolving or breaking up an occluding thrombus. A thrombolytic agent may be selected from those thrombolytic agents, which are known in the art. These include, but are not limited to, streptokinase, urokinase, prourokinase, alteplase, reteplase, anistreplase and tissue plasminogen activator (t-PA) and biologically active variants thereof. A combination of two or several thrombolytic agents may be also used.

The active ingredients are preferably administered concurrently as soon as possible, preferably within six hours, after the onset of symptoms of an acute myocardial infarction. If it is desired to avoid other medication during the thrombolytic therapy, which may be given e.g. as an intravenous bolus or infusion, levosimendan may be administered sequentially after the administration of the thrombolytic agent.

While it is preferred to administer levosimendan during or immediately after the thrombolytic therapy, it has been found that the synergistic effect of the combination is still obtained, if levosimendan administration is started not later than five days, preferably not later than three days, more preferably not later than 48 hours, from the thrombolytic therapy or, preferably, from the onset of symptoms of an acute myocardial infarction.

If desired, levosimendan administration may also be given before the thrombolytic therapy. However, in general, thrombolytic therapy should be started as soon as possible once the condition of a blocked artery has been diagnosed. Therefore, it is preferred that, in case of sequential administration, the treatment method according to the invention comprises a first step of administering an effective amount of a thrombolytic agent and a second step of administering an effective amount oflevosimendan, wherein the time period between the two treatments is not more than five days, preferably not more than three days, more preferably not more than 48 hours.

The administration routes of the active ingredients include, but are not limited to, enteral, e.g. oral or rectal, or parenteral, e.g. intravenous, intramuscular, intraperitoneal or transdermal. In the treatment of myocardial infarction, the active ingredients are preferably administered parenterally, intravenous route being particularly preferred. Single or multiple dosages may be given. Preferably, the active agents are administered via continuous infusion.

Preferably, the method comprises administering to a patient an amount of the combination, which is synergistically effective in reducing mortality of patients with myocardial infarction.

Levosimendan may be administered intravenously using an infusion rate which is from about 0.05 to 0.4 µg/kg/min. For an intravenous bolus a suitable dose is in the range from about 5 to 30 µg/kg. In the treatment of patients with acute myocardial infarction an intravenous bolus followed by continuous infusion may be needed.

Levosimedan may be administered orally to man in daily dose ranging from about 0.1 to 8 mg given once a day or divided into several doses a day, depending on the age, body weight and condition of the patient. The effective amount of levosimendan to be administered to a subject depends upon the condition to be treated, the route of administration, age, weight and the condition of the patient.

Preferred thrombolytic agents include streptokinase, urokinase, prourokinase, alteplase, reteplase, anistreplase and tissue plasminogen activator (t-PA) and biologically active variants thereof as well as any combinations thereof. The thrombolytic agent may be administered using the conventional dosage ranges for these agents, for example a daily dosage used when the agent is administered in thrombolytic therapy as a monotherapy. The range will, of course, vary depending on the thrombolytic agent employed. Examples of normal dosage ranges are as follows: urokinase - 500,000 to 6,250,000 units/patient; streptokinase - 140,000 to 2,500,000 units/patient; prourokinase - 5,000 to 100,000 units/patient; anistreplase- 10 to 100 units/patient; t-PA - 0.5 to 2.0 mg/kg body weight.

Thrombolytic therapy is typically given as an intravenous bolus alone or followed by intravenous infusion or as an infusion alone. The infusion is normally administered over a time ranging from less than one hour to about 12 hours, typically from about 1 to 3 hours. For example, the thrombolytic therapy may comprise administration of up to 10 % of the total dose as bolus injection over 1 to 5 minutes and the remaining 90 % then as a constant infusion during the next hour.

When the symptoms have been alleviated to the desired level, treatment can be stopped.

The combination may be supplemented with one or more other active ingredients, e.g. anticoagulants, or surgical methods such as angioplasty.

The active ingredients can be formulated into pharmaceutical dosage forms suitable for the treatment according to the present invention using the principles known in the art. They are given to a patient as such or preferably in combination with suitable pharmaceutical excipients in the form of tablets, granules, capsules, suppositories, emulsions, suspensions or solutions whereby the contents of the active compound in the formulation is from about 0.5 to 100 % per weight. Choosing suitable ingredients for the composition is a routine for those of ordinary skill in the art. It is evident that suitable carriers, solvents, gel forming ingredients, dispersion forming ingredients, antioxidants, colours, sweeteners, wetting compounds, release controlling components and other ingredients normally used in this field of technology may be also used.

The active ingredients may be formulated in the same pharmaceutical formulation. Preferably, such pharmaceutical composition of thrombolytic agent and levosimendan is adapted to intravenous administration. Such compositions may be prepared for storage by mixing these compounds with optional pharmaceutically acceptable carriers, excipients or stabilizers, e.g. into the form of infusion concentrates or aqueous solutions, or powders adapted to be reconstituted with sterile water or aqueous infusion vehicles for infusion.

Alternatively, the active ingredients are formulated as separate pharmaceutical dosage forms. The combination of the two pharmaceutical dosage forms may be packaged as a single medical product or kit for use in the method of the invention, optionally together with a package insert instructing to the correct use of the medical product.

Formulations suitable for intravenous administration such as injection or infusion formulation, comprise sterile isotonic solutions of the active ingredient and vehicle, preferably aqueous solutions. Typically an intravenous infusion solution of levosimendan comprises from about 0.01 to 0.1 mg/ml of levosimendan. Levosimendan composition as stored before use is preferably an infusion concentrate product, which can be reconstituted with sterile water or aqueous infusion vehicle for infusion. Levosimendan solutions suitable for use in the present invention are described e.g. in WO 01/19334.

For oral administration of levosimendan in tablet form, suitable carriers and excipients include e.g. lactose, corn starch, magnesium stearate, calcium phosphate and talc. For oral administration in capsule form, useful carriers and excipients include e.g. lactose, corn starch, magnesium stearate and talc. For controlled release oral compositions release controlling components can be used. Typical release controlling components include hydrophilic gel forming polymers such as hydroxypropylmethyl cellulose, hydroxypropyl cellulose, carboxymethyl celluloses, alginic acid or a mixture thereof; vegetable fats and oils including vegetable solid oils such as hydrogenated soybean oil, hardened castor oil or castor seed oil (sold under trade name Cutina HR), cotton seed oil (sold under the trade names Sterotex or Lubritab) or a mixture thereof; fatty acid esters such as triglycerides of saturated fatty acids or their mixtures e.g. glyceryl tristearates, glyceryl tripalmitates, glyceryl trimyristates, glyceryl tribehenates (sold under the trade name Compritol) and glyceryl palmitostearic acid ester.

Tablets can be prepared by mixing levosimendan with the carriers and excipients and compressing the powdery mixture into tablets. Capsules can be prepared by mixing levosimendan with the carriers and excipients and placing the powdery mixture in capsules, e.g. hard gelatin capsules. Typically a tablet or a capsule comprises from about 0.1 to 8 mg, more typically 0.2 to 5 mg, of levosimendan.

Thrombolytic agent compositions as used in clinical practice comprises generally water as a carrier and pharmaceutical adjuvants known in the art, i.e. isotonizing agents; acid, base or buffer substances to adjust the pH of the solution; and stabilizing agents for the thrombolytic agent. Said thrombolytic agent composition as stored before use is preferably a sterile lyophilized product, which can be reconstituted with sterile water for injection.

The concentration of the thrombolytic agent in the composition depends on the nature of the thrombolytic agent. For example, tissue plasminogen activator may be present in an amount from 20 mg to 100 mg per dosage form. The concentration of tissue plasminogen activator in a lyophilized product is typically in the range of from 1.5 to 2 % (w/w). As pH adjusting agents, phosphoric acid and optionally sodium hydroxide may be used, so that upon reconstitution with sterile water for injection, a pH of about 7.3 is reached. As stabilizing agent for the thrombolytic agent, an amino acid may be used, for example L-arginine in the case of tissue plasminogen activator. The stabilizing agent makes up the bulk of the lyophilized thrombolytic agent, typically from about 70 % to about 80 % (w/w).

Examples of preferred products according to the present invention are those wherein the thrombolytic preparation and levosimendan solution are miscible and, when mixed, form a stable formulation for up to eight hours at room temperature. The two formulations can then be stored together, but in separate containers such as vials, prefilled syringes and the like, and mixed immediately before use. A preferred container comprises the thrombolytic preparation (a) and the levosimendan solution (b) separately in a two-chamber container including means to mix both liquids. The two-chamber container is ideally a pre-filled, two-chamber syringe with bypass or similar means (e.g. a breakable seal) allowing mixing of the two separate solutions prior to administration, and which is further adapted for use with infusion devices.

Alternatively, the separate containers may be adapted to allow administration of the thrombolytic preparation and the levosimendan solution sequentially, e.g. such that levosimendan solution can be administered immediately after thrombolytic administration or e.g. after one hour from the administration of the thrombolytic agent.

Salts of levosimendan may be prepared by known methods. Pharmaceutically acceptable salts are useful as active medicaments, however, preferred salts are the salts with alkali or alkaline earth metals.

### Pharmaceutical examples.

### Example 1. Concentrate solution for intravenous infusion

| | | |
|---|---|---|
| (a) | levosimendan | 2.5 mg/ml |
| (b) | Kollidon PF12 | 10 mg/ml |
| (c) | citric acid | 2 mg/ml |
| (d) | dehydrated ethanol | ad 1 ml (785 mg) |

The concentrate solution was prepared by dissolving citric acid, Kollidon PF121 and levosimendan to dehydrated ethanol in the sterilized preparation vessel under stirring. The resulting bulk solution was filtered through a sterile filter (0.22 µm). The sterile filtered bulk solution was then aseptically filled into 8 ml and 10 ml injection vials (with 5 ml and 10 ml filling volumes) and closed with rubber closures.

The concentrate solution for intravenous infusion is diluted with an aqueous vehicle before use. Typically the concentrate solution is diluted with aqueous isotonic vehicles, such as 5 % glucose solution or 0.9 % NaCl solution so as to obtain an aqueous intravenous solution, wherein the amount of levosimendan is generally within the range of about 0.001 - 1.0 mg/ml, preferably about 0.01 - 0.1 mg/ml.

### Example 2. t-PA composition in lyophilized state

| | | |
|---|---|---|
| (a) | t-PA | 2.0 % (w/w) |
| (b) | phosphoric acid | 20 % (w/w) |
| (c) | L-arginine | 78 % (w/w) |

The ingredients were mixed, lyophilized and sterilized using standard methods. The lyophilized product comprising 20, 50 or 100 mg t-PA per dosage form (vial) is reconstituted with sterile water for injection, for example to solution having concentration of 1 mg/ml.

### Example 3. Oral levosimendan composition

| Hard gelatin capsule size 3 | |
|---|---|
| Levosimendan | 2.0 mg |
| Lactose | 198 mg |

The pharmaceutical preparation in the form of a capsule was prepared by mixing levosimendan with lactose and placing the powdery mixture in hard gelatin capsule.

### Experiments

Effect of the combination on the mortality of the patients with acute myocardial infarction

Patients who had suffered from acute myocardial infarction within five days received placebo or a 6-hour infusion of levosimendan using a bolus of 6, 12 or 24 µg/kg and subsequent infusion of 0.1, 0.2 or 0.4 µg/kg/min. Patients were divided according to whether they had received thrombolytic therapy or not. The 180 day mortality was measured. The results are shown in Table 1. It can be seen that the combination provided synergistic reduction in the mortality of patients with acute myocardial infarction.

**TABLE 1.**

| The mortality of patients with acute myocardial infarction receiving levosimendan, a thrombolytic agent or a combination thereof. | | | | |
|---|---|---|---|---|
| | Received thrombolytic | | Did not receive thrombolytic | |
| Event | Placebo (N=16) | Levosimendan (N=70) | Placebo (N=86) | Levosimendan (N=331) |
| n (%) | | | | |
| 180-day mortality | 4 (25.0 %) | 9 (12.8 %) | 28 (32.6 %) | 82 (24.8 %) |

## Claims

1. A medical product comprising, separately or together, as active ingredients a thrombolytic agent and levosimendan or a pharmaceutically acceptable salt thereof as a combined preparation.

2. A medical product as claimed in claim 1 in which the thrombolytic agent is streptokinase, urokinase, prourokinase, alteplase, reteplase, anistreplase or tissue plasminogen activator (t-PA).

3. A medical product as claimed in claim 2 in which the thrombolytic agent is t-PA.

4. A pharmaceutical composition comprising as active ingredients a thrombolytic agent and levosimendan or a pharmaceutically acceptable salt thereof.

5. Use of a thrombolytic agent and levosimendan or a pharmaceutically acceptable salt thereof as active ingredients in the manufacture of a combined preparation for simultaneous, separate or sequential administration.

6. Use of a thrombolytic agent and levosimendan or a pharmaceutically acceptable salt thereof as active ingredients in the manufacture of a medicament for reducing mortality of patients with acute myocardial infarction.

7. Use according to claim 5 or 6 in which the thrombolytic agent is streptokinase, urokinase, prourokinase, alteplase, reteplase, anistreplase or tissue plasminogen activator (t-PA).

8. Use according to claim 7 in which the thrombolytic agent is t-PA.

9. Use according to claim 8 in which the t-PA is present in an amount of from 20mg to 100 mg per dosage form.

10. Use according to claim 8 in which the active ingredients in the medicament or combined preparation are in a form suitable for intravenous administration.

## Patentansprüche

1. Medizinisches Produkt, umfassend als Wirkstoffe, getrennt voneinander oder zusammen, ein thrombolytisches Mittel und Levosimendan oder ein pharmazeutisch verträgliches Salz davon als Kombinationspräparat.

2. Medizinisches Produkt nach Anspruch 1, wobei das thrombolytische Mittel Streptokinase, Urokinase, Prourokinase, Alteplase, Reteplase, Anistreplase oder Gewebe-Plasminogenaktivator (t-PA) ist.

3. Medizinisches Produkt nach Anspruch 2, wobei das thrombolytische Mittel t-PA ist.

4. Pharmazeutische Zusammensetzung, umfassend als Wirkstoffe ein thrombolytisches Mittel und Levosimendan oder ein pharmazeutisch verträgliches Salz davon.

5. Verwendung eines thrombolytischen Mittels und von Levosimendan oder einem pharmazeutisch verträglichen Salz davon als Wirkstoffe zur Herstellung eines Kombinationspräparats zur gleichzeitigen, getrennten oder zeitlich abgestuften Verabreichung.

6. Verwendung eines thrombolytischen Mittels und von Levosimendan oder einem pharmazeutisch verträglichen Salz davon als Wirkstoffe zur Herstellung eines Medikaments zur Verringerung der Mortalität von Patienten mit akutem Myokardinfarkt.

7. Verwendung nach Anspruch 5 oder 6, wobei das thrombolytische Mittel Streptokinase, Urokinase, Prourokinase, Alteplase, Reteplase, Anistreplase oder Gewebe-Plasminogenaktivator (t-PA) ist.

8. Verwendung nach Anspruch 7, wobei das thrombolytische Mittel t-PA ist.

9. Verwendung nach Anspruch 8, wobei t-PA in einer Menge von 20 mg bis 100 mg pro Dosierungsform vorliegt.

10. Verwendung nach Anspruch 8, wobei die Wirkstoffe in dem Medikament oder dem Kombinationspräparat in einer zur intravenösen Verabreichung geeigneten Form vorliegen.

## Revendications

1. Produit médical comprenant, séparément ou ensemble, comme ingrédients actifs, un agent thrombolytique et le levosimendan ou un de ses sels pharmaceutiquement acceptables sous forme de préparation combinée.

2. Produit médical selon la revendication 1 dans lequel l'agent thrombolytique est la streptokinase, l'urokinase, la pro-urokinase, l'altéplase, le rétéplase, l'anistréplase ou l'activateur du plasminogène tissulaire (t-PA).

3. Produit médical selon la revendication 2 dans lequel l'agent thrombolytique est le t-PA.

4. Composition pharmaceutique comprenant comme ingrédients actifs un agent thrombolytique et le levosimendan ou un de ses sels pharmaceutiquement acceptables.

5. Utilisation d'un agent thrombolytique et du levosimendan ou d'un de ses sels pharmaceutiquement acceptables comme ingrédients actifs pour la fabrication d'une préparation combinée pour administration simultanée, séparée ou séquentielle.

6. Utilisation d'un agent thrombolytique et du levosimendan ou d'un de ses sels pharmaceutiquement acceptables comme ingrédients actifs pour la fabrication d'un médicament pour réduire la mortalité de patients atteints d'infarctus du myocarde aigu.

7. Utilisation selon la revendication 5 ou 6 dans laquelle l'agent thrombolytique est la streptokinase, l'urokinase, la pro-urokinase, l'altéplase, le rétéplase, l'anistréplase ou l'activateur du plasminogène tissulaire (t-PA).

8. Utilisation selon la revendication 7 dans laquelle l'agent thrombolytique est le t-PA.

9. Utilisation selon la revendication 8 dans laquelle le t-PA est présent en une quantité de 20 mg à 100 mg par forme d'administration.

10. Utilisation selon la revendication 8 dans laquelle les ingrédients actifs dans le médicament ou la préparation combinée sont sous une forme adaptée pour l'administration intraveineuse.
